# FASCICULE DE BREVET EUROPEEN

(11) **EP 1 779 842 B1**
(45) Date de publication et mention de la délivrance du brevet: **31.03.2010**
(21) Numéro de dépôt: 06291670.5
(22) Date de dépôt: 26.10.2006
(51) Int. Cl.: A61K 8/49, A61K 8/89, A61K 8/37, A61Q 5/00

(54) **Composition de soin des matières kératiniques et procédé de traitement cosmétique mettant en oeuvre ladite composition**
Zusammenstzung zum Pflegen von keratinischen Materialen und kosmetische Verwendung
Composition for treating keratinic materials and use thereof

(30) Priorité: 28.10.2005 FR 0511097
(43) Date de publication de la demande: 02.05.2007
(73) Titulaire: L'Oréal, 75008 Paris (FR)
(72) Inventeur: Masse, Virginie, 92110 Clichy (FR); Decoster, Sandrine, 95210 Saint-Gratien (FR)
(74) Mandataire: Zapalowicz, Francis

(56) Documents cités:
- FR-A- 2 804 020
- GB-A- 1 525 456
- US-A1- 2004 197 287

## Description

La présente invention est relative à une composition cosmétique améliorée pour le soin des matières kératiniques, en particulier le lavage et/ou le traitement cosmétique des cheveux et/ou de la peau, et qui comprend au moins une silicone, au moins un mono ou diester d'acide gras et d'éthylèneglycol ou de polyéthylèneglycol, ainsi qu'un ester d'acide gras et de sorbitan oxyéthyléné.

La présente invention concerne également un procédé de traitement cosmétique mettant en oeuvre ladite composition.

De nombreuses compositions de lavage des matières kératiniques ont été décrites dans l'art antérieur.

Ainsi, la demande de brevet FR 2 848 829 décrit des compositions détergentes destinées essentiellement au lavage des cheveux, qui présentent des propriétés cosmétiques améliorées en particulier en termes de démêlage, lissage, souplesse, maléabilité et douceur des cheveux.

Ces compositions comprennent au moins un tensioactif amphotère alcoylamphohydroxyalkylsulfonate, et au moins une silicone choisie parmi les silicones non organomodifiées de viscosité allant de 500 mm²/s à 1 000 000 mm²/s et les silicones organomodifiées.

Le brevet CA 1 201 952 décrit des compositions tensioactives destinées à la formulation de compositions cosmétiques, en particulier des shampooings, dont le pouvoir moussant est amélioré. Ces compositions comprennent
- de 3,75 à 15 % en poids d'un tensioactif anionique tel qu'un lauryl éther sulfate,
- de 1 à 4,20 % en poids d'un tensioactif amphotère tel qu'une alkyl amido bétaïne,
- de 0,7 à 3% en poids d'un tensioactif non ionique tel qu'un sorbitan polyoxyéthyléné, et
- de 0,1 à 4 % en poids d'un « savon » choisi parmi les acides gras, les alkyle isethionates, les alkyle taurides et les alkyle sarcosides.

La demande de brevet US 2004/0197287 décrit des shampooings antipelliculaires dont les propriétés détergentes et antipelliculaires sont améliorées. Ces compositions comprennent :
- de 5 à 50% en poids d'un tensioactif détergent,
- de 0,1 à 4% en poids d'un agent antipelliculaire,
- de 0,1 à 50% en poids d'un ester d'acide gras et de sorbitan polyoxyéthyléné, en particulier l'un ou l'autre des produits commercialisés sous les noms Tween 40, Tween 60, Tween 61 et Tween 85 par la société Uniqema, et
- au moins 20% en poids d'eau.

Enfin, la demande de brevet WO 97/33561 décrit des compositions nettoyantes pour les cheveux et la peau, qui présentent un faible degré d'irritation oculaire. Ces compositions comprennent :
- de 5 à 20 % en poids d'un mélange de tensioactifs dont un tensioactif non ionique, un tensioactif amphotère et un tensioactif anionique, et
- de 0,01 à 3% en poids d'un agent humectant, tel que notamment un polyol cationique.

Comme tensioactif non ionique, sont employés entre autres des dérivés polyoxyéthylénés d'ester d'acide gras et de polyol, comportant de 10 à 120 unités oxyéthylène.

Toutefois, les compositions décrites dans l'art antérieur présentent certaines insuffisances. En particulier, les shampooings les plus performants peuvent provoquer des picotements dans l'oeil lorsque le produit dilué coule dans la sphère oculaire, ce qui arrive fréquemment chez les enfants. Par ailleurs, bon nombre de ces shampooings provoquent, chez les personnes présentant une peau sensible, des réactions d'inconfort telles que des rougeurs, des démangeaisons, des picotements.

Les compositions douces proposées dans l'art antérieur présentent à l'inverse des qualités d'usage insuffisantes (viscosités inadéquates, démarrage et qualité de mousse médiocres) et des propriétés cosmétiques également insuffisantes notamment en termes de douceur et, pour les cheveux, en plus en terme de démêlage et de lissage.

La Demanderesse a maintenant découvert, de manière surprenante, qu'en associant, en présence d'une silicone, un agent nacrant et un agent tensioactif particuliers, il est possible de formuler des compositions cosmétiques particulièrement douces et non agressives, avec de bonnes qualités d'usage notamment en terme de viscosité, et qui présentent néanmoins d'excellentes propriétés tant détergentes que cosmétiques.

Ainsi, les compositions selon l'invention permettent de diminuer les réactions d'inconfort au niveau de la peau et du cuir chevelu, et possèdent un excellent niveau de tolérance oculaire. Parallèlement, elles possèdent des propriétés nettoyantes d'un bon niveau ainsi que d'excellentes propriétés cosmétiques, notamment un bon niveau de douceur et, en ce qui concerne les cheveux, outre ce bon niveau de douceur, plus de démêlage et de lissage. De plus, elles présentent un aspect nacré particulièrement esthétique.

La présente invention a donc pour objet une composition cosmétique pour le soin des matières kératiniques comprenant, dans un milieu aqueux :
- au moins une silicone,
- au moins un mono ou diester d'acide gras et d'éthylèneglycol ou de polyéthylèneglycol, et
- du mono-laurate de sorbitan oxyéthyléné à 4 OE.

Un autre objet de l'invention est un procédé de traitement cosmétique mettant en oeuvre ladite composition.

D'autres objets et caractéristiques, aspects et avantages de l'invention apparaîtront encore plus clairement à la lecture de la description et des exemples qui suivent.

Dans le présent exposé, et de manière bien connue en soi, on désigne par « composé à X OE » un composé oxyéthyléné comprenant X unités oxyéthylène par molécule.

On désigne de plus par « composé en CY » un composé comprenant Y atomes de carbone.

Les silicones utilisables dans la composition selon l'invention peuvent être solubles ou insolubles dans ladite composition.

Elles peuvent en particulier comprendre un ou plusieurs polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les polyorganosiloxanes sont définis plus en détail dans l'ouvrage de Walter NOLL "Chemistry and Technology of Silicones" (1968) Academic Press. Ils peuvent être volatiles ou non volatiles.

Ainsi, la composition selon l'invention peut comprendre au moins un polyorganosiloxane volatile, choisi parmi ceux possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 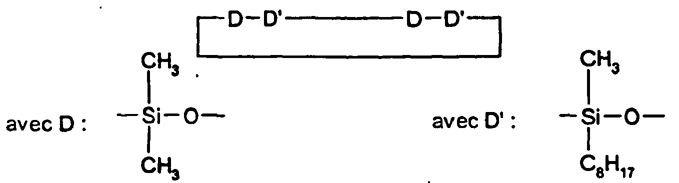 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

De même, la composition selon l'invention peut comprendre au moins un polyorganosiloxane non volatile, choisi parmi les polyalkylsiloxanes, les polyarylsiloxanes, les polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyl-diphényl-siloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHONE POULENC;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane,
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Peuvent également être employés des mélanges de silicones tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements ammonium quaternaires comme les produits commercialisés sous les dénominations ABILQUAT 3272 et ABILQUAT 3474 par la société GOLDSCHMIDT ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255".
- des groupements hydroxyacylamino, comme les polyorganosiloxanes décrits dans la demande EP 342 834. On peut citer, par exemple, le produit Q2-8413 de la société DOW CORNING.

Les silicones particulièrement préférées dans l'invention sont les polydiméthylsiloxanes tels que les polydiméthylsiloxanes à groupements terminaux triméthylsilyle, ou les polydiméthylsiloxanes à groupements terminaux hydroxydiméthylsilyle, et les silicones aminées.

La composition cosmétique selon l'invention contient avantageusement au moins 0,1 % en poids de silicone(s), par rapport au poids total de la composition. De préférence, elle contient de 0,1 à 20 % en poids de silicone(s), plus préférentiellement de 0,5 à 20% en poids, encore plus préférentiellement de 0,7 à 10 % en poids, et mieux encore de 1 à 5 % en poids par rapport au poids total de la composition.

La composition selon l'invention comprend en outre au moins un mono ou diester d'acide gras et d'éthylèneglycol ou de polyéthylèneglycol.

Le (les) acide(s) gras est (sont) avantageusement en C₈ à C₃₀, de préférence en C₁₂ à C₂₄.

Lorsque l'ester comprend un groupement polyéthylèneglycol, ce dernier comprend de préférence de 2 à 10 unités éthylèneglycol, et plus préférentiellement de 2 à 5 unités éthylèneglycol.

De préférence, l'ester est un mono ou un diester d'acide gras en C12 à C24 et d'éthylèneglycol. De manière encore plus préférée, l'ester est un mono ou distéarate d'éthylèneglycol. Encore plus préférentiellement, l'ester est le distéarate d'éthylèneglycol

La composition selon l'invention contient préférentiellement au moins 0,5% en poids de mono ou diester(s) d'acide gras et d'éthylèneglycol ou de polyéthylèneglycol, par rapport au poids total de la composition. De préférence, elle contient de 0,5 à 10 % en poids, plus préférentiellement de 0,8 à 10 % en poids, et encore plus préférentiellement de 1 à 5 % en poids de mono ou diester(s) d'acide gras et d'éthylèneglycol ou de polyéthylèneglycol, par rapport au poids total de la composition.

La composition objet de la présente invention comprend .

du mono-laurate de sorbitan oxyéthylène à 4 OE. Ce composé est également connu sous le nom de polysorbate 21. Il est, entre autres, commercialisé sous la dénomination TWEEN 21 par la société UNIQEMA.

La composition selon l'invention comprend préférentiellement au moins 0,5 % en poids de mono-laurate de sorbitan oxyéthyléné à 4 OE, par rapport au poids total de la composition. De préférence, elle comprend de 0,5 à 10 % en poids de mono-laurate de sorbitan oxyéthyléné à 4 OE, plus préférentiellement de 2 à 9 % en poids, encore plus préférentiellement de 4 à 8 % en poids, par rapport au poids total de la composition.

Le mono-laurate de sorbitan oxyéthyléné à 4 OE peut être utilisé seul, ou en mélange avec un ou plusieurs autres dérivés oxyéthylénés du sorbitan. Par exemple, il est avantageux d'employer également dans les compositions selon l'invention au moins un monoester d'acide gras en C₈ à C₂₄ et de sorbitan oxyéthyléné comprenant de 15 à 50 unités oxyéthylène.

De préférence, ledit monoester est un monoester d'acide gras en C₈ à C₂₄ et de sorbitan oxyéthyléné comprenant 20 unités oxyéthylène.

De manière encore plus préférée, ledit monoester est le mono-laurate de sorbitan oxyéthylène à 20 OE.

La composition selon l'invention comprend préférentiellement de 0,1 à 10 % en poids de monoester d'acide gras en C8 à C24 et de sorbitan oxyéthyléné comprenant de 15 à 50 unités oxyéthylène, plus préférentiellement de 0,5 à 5 % en poids, par rapport au poids total de la composition.

Les compositions selon l'invention peuvent se présenter par exemple sous la forme de gels douche, de shampooings, de compositions à appliquer avant ou après un shampooing, ces dernières se présentant sous la forme d'une lotion plus ou moins épaissie, d'un gel ou d'une émulsion.

Les compositions de l'invention peuvent également comprendre un ou plusieurs tensioactifs anioniques.

Les tensioactifs anioniques pouvant être utilisés dans les compositions selon l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyllactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Lorsqu'ils sont présents, la quantité du ou des tensioactifs anioniques est de préférence comprise dans l'intervalle allant de 0,5 à 50 % en poids, mieux encore de 4 à 20% en poids par rapport au poids total de la composition.

La composition selon l'invention peut également comprendre, en plus du mono-laurate de sorbitan oxyéthyléné à 4 OE, un ou plusieurs autres tensioactifs non-ioniques additionnels, différent(s) de ce(s) dernier(s).

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels est de préférence comprise dans l'intervalle allant de 0,01 à 10 % en poids, mieux encore de 0,05 à 5 % en poids par rapport au poids total de la composition.

Les compositions de l'invention peuvent également comprendre un ou plusieurs tensioactifs amphotères ou zwittérioniques.

Les agents tensioactifs amphotères ou zwittérioniques utilisables dans les compositions selon l'invention peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer également les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (I) et (II) :

Rₐ-CONHCH₂CH₂-N(R_{b})(R_{c})(CH₂COO⁻) (I)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (II)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques est de préférence comprise dans l'intervalle allant de 0,1 à 10 % en poids, mieux encore de 0,5 à 8 % en poids par rapport au poids total de la composition.

La composition selon l'invention présente de préférence une teneur totale en tensioactifs anioniques, non-ioniques, amphotères et zwittérioniques comprise dans l'intervalle allant de 4 à 50 % en poids, mieux encore de 4 à 20 % en poids, par rapport au poids total de la composition.

Les compositions selon la présente invention peuvent comprendre en outre au moins un tensioactif cationique.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Lorsque les tensioactifs cationiques sont présents, leur quantité est de préférence comprise dans l'intervalle allant de 0,01 à 10 % en poids, mieux encore de 0,05 à 5 % en poids, et encore plus préférentiellement de 0,3 à 3 % en poids par rapport au poids total de la composition cosmétique.

De manière avantageuse, la composition selon l'invention comprend au moins un tensioactif anionique et au moins un tensioactif amphotère ou zwittérionique.

Les compositions selon la présente invention peuvent comprendre en outre au moins un polymère cationique.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont une masse moléculaire moyenne en poids supérieure à 10⁵, de préférence supérieure à 10⁶ et mieux encore comprise entre 10⁶ et 10⁸.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides acryliques ou méthacryliques et comportant au moins un des motifs de formules suivantes: 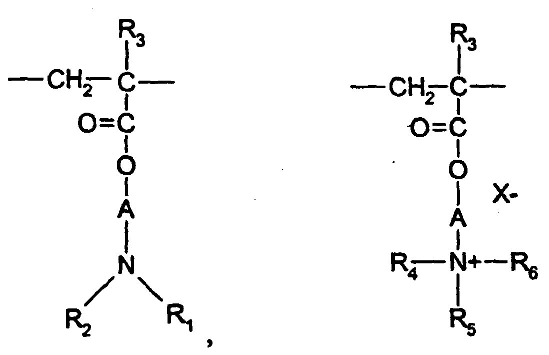 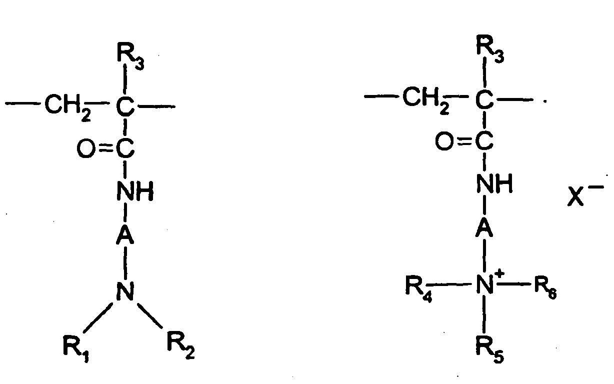 dans lesquelles:
   R₁ et R₂, identiques ou différents, représentent un atome d'hydrogène ou un groupe alkyle ayant de 1 à 6 atomes de carbone, et de préférence un groupe méthyle ou éthyle ;
   R₃, identiques ou différents, désignent un atome d'hydrogène ou un groupe CH₃ ;
   les symboles A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone ;
   X désigne un anion dérivé d'un acide minéral ou organique tel qu'un anion méthosulfate ou un halogénure comme le chlorure ou le bromure.
   Les copolymères de la famille (1) peuvent contenir en outre un ou plusieurs motifs dérivant de comonomères pouvant être choisis dans la famille des acrylamides, méthacrylamides, diacétone-acrylamides, acrylamides et méthacrylamides substitués sur l'atome d'azote par des groupes alkyle inférieur (C₁-C₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de diméthyle,
   - les copolymères d'acrylamide et de chlorure de méthacryloyloxyéthyltriméthylammonium décrits, par exemple, dans la demande de brevet EP-A-080976,
   - les copolymères d'acrylamide et de méthosulfate de méthacryloyloxyéthyltriméthylammonium,
   - les copolymères vinylpyrrolidone/acrylate ou méthacrylate de dialkylaminoalkyle quaternisés ou non. Ces polymères sont décrits en détail dans les brevets français 2 077 143 et 2 393 573,
   - les terpolymères méthacrylate de diméthylaminoéthyle/vinylcaprolactame/vinylpyrrolidone,
   - les copolymères vinylpyrrolidone/méthacrylamidopropyl-di-méthylamine, et
   - les copolymères vinylpyrrolidone/méthacrylamide de diméthylaminopropyle quaternisé.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.
   Les produits commercialisés répondant à cette définition sont plus particulièrement les produits vendus sous la dénomination "Celquat® L 200" et "Celquat® H 100" par la Société National Starch.
(4) Les polysaccharides cationiques non cellulosiques décrits dans les brevets US 3 589 578 et 4 031 307 tels que les gommes de guar contenant des groupements cationiques trialkylammonium. On utilise, par exemple, des gommes de guar modifiées par un sel, par exemple le chlorure de 2,3-époxypropyltriméthylammonium.
   De tels produits sont commercialisés notamment sous les dénominations commerciales de JAGUAR® C13 S, JAGUAR® C15, JAGUAR® C17 ou JAGUAR® C162 par la société MEYHALL.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(6) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alkylés ou s'ils comportent une ou plusieurs fonctions amines tertiaires, quaternisées. De tels polymères sont notamment décrits dans les brevets français 2 252 840 et 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
   Parmi ces dérivés, on peut citer plus particulièrement les polymères acide adipique/diméthylaminohydroxypropyl-diéthylène-triamine.
(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(9) Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 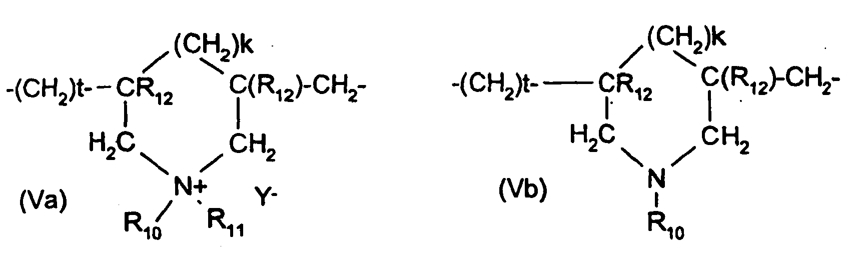 dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou alors R₁₀ et R₁₁ peuvent désigner conjointement avec l'atome d'azote auquel ils sont rattachés, des groupements hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT® 100" par la société CALGON (et ses homologues de faibles masses moléculaires moyenne en poids) et les copolymères de chlorure de diallyldiméthylammonium et d'acrylamide commercialisés sous la dénomination "MERQUAT® 550".
(10) Les polymères de diammonium quaternaire contenant des motifs récurrents répondant à la formule (VI) : 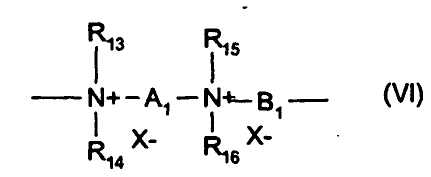 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-E ou -CO-NH-R₁₇-E où R₁₇ est un groupe alkylène et E un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-E'-OC-(CH₂)ₙ-

      dans lequel E' désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-
         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH-.
   De préférence, X- est un anion tel que le chlorure ou le bromure.
   Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
   On peut utiliser plus particulièrement les polymères qui sont constitués de motifs récurrents répondant à la formule : 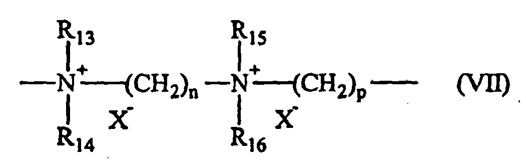 dans laquelle R₁₃, R₁₄, R₁₅ et R₁₆, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(11) Les polymères de polyammonium quaternaire constitués de motifs de formule (VIII) : 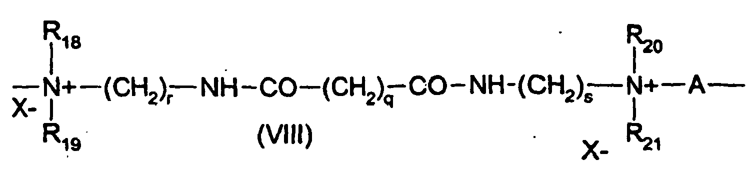 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.
   De tels composés sont notamment décrits dans la demande de brevet EP-A-122 324.
(12) Les polymères quaternaires de vinylpyrrolidone et de vinylimidazole.
(13) Les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose comportant des groupements ammonium quaternaires tels que les produits vendus sous la dénomination "JR 400" par la Société UNION CARBIDE CORPORATION, les cyclopolymères cationiques, en particulier les homopolymères ou copolymères de chlorure de diméthyldiallylammonium, vendus sous les dénominations MERQUAT® 100, MERQUAT® 550 et MERQUAT® S par la société CALGON, les gommes de guar modifiées par un sel de 2,3-époxypropyl-triméthylammonium, les polymères quaternaires de vinylpyrrolidone et de vinylimidazole.

Lorsque le(s) polymère(s) cationique(s) est (sont) présent(s), la composition selon l'invention comprend de 0,01 à 10 % en poids de polymère(s) cationique(s), mieux encore de 0,1 à 5 % en poids, et encore plus préférentiellement de 0,1 à 2 % en poids, par rapport au poids total de la composition.

La composition selon l'invention peut comprendre en outre un ou plusieurs agent(s) antipelliculaire(s). Comme agents antipelliculaires, peuvent être employés par exemple des composés tels que la piroctone olamine, la pyrithione de zinc, l'acide salicylique, le disulfure de sélénium, et leurs mélanges.

La composition comprend alors de 0,001 à 10 % en poids d'agent(s) antipelliculaire(s), de préférence de 0,1 à 5 % en poids, encore plus préférentiellement de 0,2 à 2 % en poids, par rapport au poids total de la composition.

Le milieu aqueux est constitué d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, l'hexylèneglycol, le propylèneglycol, les polyéthylèneglycols, et leurs mélanges.

Le pH des compositions selon l'invention est généralement inférieur à 8,5, et de préférence compris dans l'intervalle allant de 4 à 7.

La composition selon l'invention peut comprendre en outre un ou plusieurs additifs classiques bien connus dans la technique, tels que les agents antichute, les agents oxydants, les céramides et pseudo-céramides, les vitamines et pro-vitamines dont le panthénol, les huiles végétales, animales, minérales ou synthétiques, les cires, les céramides et pseudo-céramides, les filtres solaires, les pigments minéraux ou organiques, colorés ou non colorés, les colorants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

L'homme de métier veillera à choisir les éventuels additifs et leurs quantités de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

La composition selon l'invention peut être utilisée notamment comme composition de traitement ou de soin cosmétique des matières kératiniques.

Par matières kératiniques, on comprend les cheveux, les cils, les sourcils, la peau, les ongles, les muqueuses ou le cuir chevelu, et plus particulièrement les cheveux.

En particulier, la composition selon l'invention peut être utilisée comme shampooing ou composition à appliquer avant ou après un shampooing.

Un autre objet de l'invention est un procédé de traitement cosmétique, qui comprend l'application sur les cheveux d'une quantité efficace d'une composition cosmétique telle que décrite ci-dessus.

Selon un mode de mise en oeuvre préféré, un tel procédé consiste à appliquer sur les cheveux une quantité efficace de la composition cosmétique, à éventuellement rincer après un éventuel temps de pause.

Lorsque l'on applique la composition selon l'invention sous forme d'une lotion ou d'une crème avant ou après shampooing, on la laisse éventuellement pauser sur les cheveux pendant environ 1/2 minute à 5 minutes, puis on rince éventuellement à l'eau.

Les exemples suivants sont donnés à titre illustratif de la présente invention, et ne sauraient en limiter la portée.

### EXEMPLES

Dans les exemples suivants, toutes les quantités sont indiquées en pour cent en poids de matière active (MA) par rapport au poids total de la composition, sauf indication contraire.

### Exemple 1 :

Une première composition de shampooing, conforme à l'invention, est préparée à partir des ingrédients indiqués dans le tableau ci-dessous (en g de MA).

| Composition | 1 |
|---|---|
| lauryl éther sulfate de sodium à 2,2 OE en solution aqueuse à 70% MA (Texapon N702 de Cognis) | 15,3 |
| mono-laurate de sorbitan oxyéthylène à 4 OE (Tween 21 de Uniqema) | 6 |
| cocoyl amidopropyl bétaïne / monolaurate de glycérine en solution aqueuse 25 / 75 en poids (Tégobétaïne de Goldschmidt) | 1,6 |
| distéarate d'éthylène glycol (Tegin BL 315 de Goldschmidt) | 2 |
| N-cocoyl amidoéthyl, N-éthoxycarboxyméthyl glycinate de sodium (Miranol C2M conc de Rhodia) | 0,8 |
| poly diméthylsiloxane (PM 250 000) (Mirasil DM 500 000 de Rhodia) | 2 |
| piroctone olamine (Octopirox de Clariant) | 0,5 |
| benzoate de sodium | 0,5 |
| parfum | 0,5 |
| mélange de p-hydroxybenzoates de méthyle, butyle, éthyle, propyle, isobutyle (7/57/22/14) (Nipastat de Nipa) | 0,5 |
| acide salicylique en poudre | 0,2 |
| polymère carboxyvinylique synthétisé dans un mélange acétate d'éthyle / cyclohexane (Carbopol 980 de Noveon) | 0,2 |
| p-hydroxybenzoate de méthyle | 0,03 |
| Eau qsp | 100% |

La composition de shampooing détaillée ci-dessus s'est avérée présenter une excellente tolérance vis-à-vis du cuir chevelu. En particulier, l'on a observé très peu de réactions d'inconfort. En outre, cette composition présente une excellente tolérance oculaire.

Enfin, cette composition présente de bonnes qualités d'usage, ainsi que de remarquables propriétés cosmétiques de douceur, de démêlage et de lissage.

### Exemples 2 à 4 :

Trois compositions de shampooing, conformes à l'invention, sont préparées à partir des ingrédients indiqués dans le tableau ci-dessous.

| Compositions | 2 | 3 | 4 |
|---|---|---|---|
| lauryl éther sulfate de sodium (2,2 OE) en solution aqueuse (Texapon N702 de Cognis) | 11,9 | 15,8 | 15,5 |
| cocoyl amidopropyl bétaïne / monolaurate de glycérine en solution aqueuse 25 / 75 (Tégobétaïne de Goldschmidt) | 1,1 | 1,7 | - |
| mono-laurate de sorbitan oxyéthylène à 4 OE (Tween 21 de Uniqema) | 6 | 6 | 6 |
| mono-laurate de sorbitan oxyéthylène à 20 OE (Tween 20 de Uniqema) | - | - | 1 |
| distéarate d'éthylène glycol (Tegin BL 315 de Goldschmidt) | 2 | 2 | 2 |
| N-cocoyl amidoéthyl, N-éthoxycarboxyméthyl glycinate de sodium (Miranol C2M conc de Rhodia) | 0,8 | 0,8 | 3,1 |
| poly diméthylsiloxane (Mirasil DM 500 000 de Rhodia) | 1,5 | 2,7 | 2,7 |
| hexylène glycol (2 méthyl-2,4 pentanediol) | 1 | - | - |
| monoisopropanolamide d'acides de coprah (Empilan cis de Huntsman) | 0,8 | - | - |
| chlorure de sodium | 0,5 | - | - |
| benzoate de sodium | 0,5 | 0,5 | 0,5 |
| parfum | 0,5 | 0,5 | 0,5 |
| mélange de p-hydroxybenzoates de méthyle, butyle, éthyle, propyle, isobutyle (7/57/22/14) (Nipastat de Nipa) | 0,5 | 0,5 | 0,5 |
| hydroxyéthyl cellulose quaternisée par du chlorure de 2,3 époxypropyl triméthyl ammonium (Celquat SC 240C de National Starch) | 0,4 | - | - |
| acide salicylique en poudre | 0,2 | 0,2 | 0,2 |
| polymère carboxyvinylique synthétisé dans un mélange acétate d'éthyle / cyclohexane (Carbopol 980 de Noveon) | 0,2 | 0,2 | 0,15 |
| p-hydroxybenzoate de méthyle, sel de sodium (Nipagin M sodium de Clariant) | 0,03 | 0,03 | 0,03 |
| chlorure d'hydroxypropyl guar triméthylammonium (Jaguar C13S de Rhodia) | - | 0,05 | 0,2 |
| Eau qsp | 100% | 100% | 100% |

Les compositions de shampooing détaillées ci-dessus se sont également avérées présenter une excellente tolérance vis-à-vis du cuir chevelu. En particulier, l'on a observé très peu de réactions d'inconfort. En outre, ces compositions présentent une excellente tolérance oculaire.

Enfin, ces compositions présentent de très bonnes qualités d'usage, ainsi que de remarquables propriétés cosmétiques de douceur, de démêlage et de lissage.

### Exemples 5 à 7 :

Trois compositions de shampooings sont préparées à partir des ingrédients indiqués dans le tableau ci-dessous. La composition 7 est conforme à l'invention, les compositions 5 et 6 sont présentées à titre comparatif.

| Compositions | 5 | 6 | 7 |
|---|---|---|---|
| lauryl éther sulfate de sodium (2,2 OE) en solution aqueuse (Texapon N702 de Cognis) | 11,7 | 11,7 | 11,7 |
| cocoyl amidopropyl bétaïne / monolaurate de glycérine en solution aqueuse 25 / 75 (Tégobétaïne de Goldschmidt) | 1 | 1 | 1 |
| mono-laurate de sorbitan oxyéthylène à 4 OE (Tween 21 de Uniqema) | - | - | 8 |
| mono-laurate de sorbitan oxyéthylène à 20 OE (Tween 20 de Uniqema) | - | 8 | - |
| mono-stéarate de sorbitan oxyéthylène à 4 OE (Tween 61 de Uniqema) | 8 | - | - |
| distéarate d'éthylène glycol (Tegin BL 315 de Goldschmidt) | 2 | 2 | 2 |
| N-cocoyl amidoéthyl, N-éthoxycarboxyméthyl glycinate de sodium (Miranol C2M conc de Rhodia) | 0,8 | 0,8 | 0,8 |
| poly diméthylsiloxane (Mirasil DM 500 000 de Rhodia) | 1 | 1 | 1 |
| monoisopropanolamide d'acides de coprah (Empilan cis de Huntsman) | 0,3 | 0,3 | 0,3 |
| benzoate de sodium | 0,5 | 0,5 | 0,5 |
| parfum | 0,5 | 0,5 | 0,5 |
| mélange de p-hydroxybenzoates de méthyle, butyle, éthyle, propyle, isobutyle (7/57/22/14) (Nipastat de Nipa) | 0,5 | 0,5 | 0,5 |
| hydroxyéthyl cellulose quaternisée par du chlorure de 2,3 époxypropyl triméthyl ammonium (Celquat SC 240C de National Starch) | 0,4 | 0,4 | 0,4 |
| acide salicylique en poudre | 0,2 | 0,2 | 0,2 |
| polymère carboxyvinylique synthétisé dans un mélange acétate d'éthyle / cyclohexane (Carbopol 980 de Noveon) | 0,2 | 0,2 | 0,2 |
| p-hydroxybenzoate de méthyle, sel de sodium (Nipagin M sodium de Clariant) | 0,03 | 0,03 | 0,03 |
| Eau qsp | 100% | 100% | 100% |

La composition 7 selon l'invention présente en particulier, par rapport aux compositions comparatives 5 et 6, des avantages en termes de mise en oeuvre, et de viscosité.

En premier lieu, le mono-stéarate de sorbitan oxyéthylène à 4 OE employé dans la composition comparative 5 doit être fondu avant son incorporation dans le shampooing, ce qui rend la préparation de ce dernier plus difficile. A l'inverse, le mono-laurate de sorbitan oxyéthylène à 4 OE employé dans la composition 7 conforme à l'invention est liquide à température ambiante. Il peut donc être incorporé dans le shampooing à température ambiante, d'où un gain substantiel en termes de temps et d'énergie.

En second lieu les viscosités des compositions comparatives 5 et 6 ne sont pas adéquates, par rapport à la consistance habituelle des shampooings.

En effet, la composition 6 présente un temps d'écoulement à 25°C de 3 s CF 10, ce qui correspond à une viscosité trop basse, tandis que la composition 7 présente un temps d'écoulement à 25°C de 60 s CF 10, ce qui correspond à une viscosité plus appropriée pour un shampooing.

Enfin, la composition 5 présente une viscosité beaucoup trop élevée.

Ainsi, la composition 7 conforme à l'invention présente des qualités d'usage supérieures aux compositions comparatives : elle n'est ni trop épaisse, ni trop liquide, permettant ainsi une meilleure répartition du produit sur les cheveux avec une bonne qualité de mousse.

## Revendications

1. Composition cosmétique pour le soin des matières kératiniques comprenant, dans un milieu aqueux :
- au moins une silicone,
- au moins un mono ou diester d'acide gras et d'éthylèneglycol ou de polyéthylèneglycol, et
- du mono-laurate de sorbitan oxyéthyléné à 4 OE.

2. Composition selon la revendication précédente, **caractérisée en ce qu'**elle comprend au moins 0.5% en poids de mono-laurate de sorbitan oxyéthyléné à 4 OE par rapport au poids total de la compositions.

3. Composition selon la revendication 2, **caractérisée en ce qu'**elle comprend de à 1 % en poids de mono-laurate de sorbitan oxyéthyléné à 4 OE, plus préférentiellement de 2 à 9 % en poids, par rapport au poids total de la composition.

4. Composition selon l'une quelconque des revendications précédentes, **caractérisée en ce que** la silicone comprend un ou plusieurs polyorganosiloxanes insolubles dans ladite composition.

5. Composition selon la revendication précédente, **caractérisée en ce qu'**elle comprend au moins un polyorganosiloxane volatile, choisi parmi ceux possédant un point d'ébullition compris entre 60° C et 260° C, et plus particulièrement parmi :
(i) les silicones cycliques comportant de 3 à 7 atomes de silicium, et
(ii) les silicones volatiles linéaires ayant 2 à 9 atomes de silicium et possédant une viscosité inférieure ou égale à 5.10⁻⁶m²/s à 25°C.

6. Composition selon la revendication 4, **caractérisée en ce qu'e**lle comprend au moins un polyorganosiloxane non volatile, choisi parmi les polyalkylsiloxanes, les polyarylsiloxanes, les polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non silicosé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non silicones, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

7. Composition selon la revendication 6, **caractérisée en ce que** les polyalkylsiloxanes sont choisis parmi les polydiméthylsiloxanes.

8. Composition selon l'une quelconque des revendications précédentes, **caractérisée en ce qu'**elle contient au moins 0,1 % en poids de silicone(s), par rapport au poids total de la composition.

9. Composition selon la revendication 6, **caractérisée en ce qu'**elle contient de 0,1 à 20 % en poids de silicone(s), plus préférentiellement de 0,5 à 20% en poids, encore plus préférentiellement de 0,7 à 10 % en poids, par rapport au poids total de la composition.

10. Composition selon l'une quelconque des revendications précédentes, **caractérisée en ce que** ledit mono ou diester d'acide gras et d'éthylèneglycol ou de polyéthylèneglycol comprend un groupement polyéthylèneglycol, ce dernier comprenant de 2 à 10 unités éthylèneglycol.

11. Composition selon l'une quelconque des revendications 1 à 9, **caractérisée en ce que** ledit mono ou diester d'acide gras et d'éthylèneglycol ou de polyéthylèneglycol est un mono ou un diester d'acide gras en C₁₂ à C₂₄ et d'éthylèneglycol.

12. Composition selon la revendication 11, **caractérisée en ce que** ledit mono ou diester d'acide gras et d'éthylèneglycol ou de polyéthylèneglycol est le distéarate d'éthylèneglycol.

13. Composition selon l'une quelconque des revendications précédentes, **caractérisée en ce qu'**elle contient au moins 0,5% en poids de mono ou diester(s) d'acide gras et d'éthylèneglycol ou de polyéthylèneglycol, par rapport au poids total de la composition.

14. Composition selon la revendication précédente, **caractérisée en ce qu'**elle contient de 0,5 à 10 % en poids, plus préférentiellement de 0,8 à 10 % en poids, et encore plus préférentiellement de 1 à 5 % en poids de mono ou diester(s) d'acide gras et d'éthylèneglycol ou de polyéthylèneglycol, par rapport au poids total de la composition.

15. Composition selon l'une quelconque des revendications précédentes, **caractérisée en ce qu'**elle comprend également au moins un monoester d'acide gras en C8 à C24 et de sorbitan oxyéthyléné comprenant de 15 à 50 unités oxyéthylène.

16. Composition selon la revendication précédente, **caractérisée en ce que** ledit monoester est un monoester d'acide gras en C8 à C24 et de sorbitan oxyéthyléné comprenant 20 unités oxyéthylène, de préférence le mono-laurate de sorbitan oxyéthylène à 20 OE.

17. Composition selon l'une quelconque des revendications 15 et 16, **caractérisée en ce qu'**elle comprend de 0,1 à 10 % en poids de monoester d'acide gras en C8 à C24 et de sorbitan oxyéthyléné comprenant de 15 à 50 unités oxyéthylène, de préférence de 0,5 à 5 % en poids, par rapport au poids total de la composition.

18. Composition selon l'une quelconque des revendications précédentes, **caractérisée en ce qu'**elle comprend en outre au moins un tensioactif anionique et/ou non ionique et/ou amphotère.

19. Composition selon la revendication 18, **caractérisée en ce qu'**elle comprend au moins un tensioactif anionique et au moins un tensioactif amphotère ou zwittérionique.

20. Composition selon l'une quelconque des revendications 18 ou 19, **caractérisée en ce que** le tensioactif anionique est choisi parmi les alkylsulfates, les alkyléthersulfates et leurs mélanges.

21. Composition selon l'une quelconque des revendications 18 à 20, **caractérisée en ce que** la quantité du ou des tensioactifs anioniques est comprise dans l'intervalle allant de 0,5 à 50 % en poids, mieux encore de 4 à 20% en poids par rapport au poids total de la composition.

22. Composition selon l'une quelconque des revendications 18 à 20, **caractérisée en ce que** le tensioactif amphotère ou zwittérionique est choisi parmi les (alkyl en C₈₋₂₀)-bétaïnes, les (alkyl en C₈₋₂₀)-amido(alkyl en C₆₋₈)bétaïnes et leurs mélanges.

23. Composition selon l'une quelconque des revendications précédentes, **caractérisée en ce qu'**elle comprend en outre au moins un tensioactif cationique.

24. Composition selon l'une quelconque des revendications précédentes, **caractérisée en ce qu'**elle comprend en outre au moins un polymère cationique.

25. Composition selon la revendication 24, **caractérisée en ce qu'**elle comprend de 0,01 à 10 % en poids de polymère(s) cationique(s), de préférence de 0,1 à 5 % en poids, par rapport au poids total de la composition

26. Composition selon l'une quelconque des revendications précédentes, **caractérisée en ce qu'**elle comprend en outre un ou plusieurs agent(s) antipelliculaire(s).

27. Composition selon la revendication 26, **caractérisée en ce qu'**elle comprend de 0,001 à 10 % en poids d'agent(s) antipelliculaire(s), de préférence de 0,1 à 5 % en poids, par rapport au poids total de la composition.

28. Composition selon l'une quelconque des revendications précédentes, **caractérisée en ce que** le milieu aqueux est constitué d'eau ou d'un mélange d'eau et d'au moins un solvant cosmétiquement acceptable.

29. Composition selon la revendication 28, **caractérisée en ce que** le solvant est choisi parmi les alcools inférieurs en C₁-C₄ et les polyols.

30. Composition selon l'une quelconque des revendications précédentes, **caractérisée en ce qu'**elle comprend en outre un ou plusieurs additifs classiques, tels que les agents antichute, les agents oxydants, les céramides et pseudo-céramides, les vitamines et provitamines dont le panthénol, les huiles végétales, animales, minérales ou synthétiques, les cires, les céramides et pseudo-céramides, les filtres solaires, les pigments minéraux ou organiques, colorés ou non colorés, les colorants, les agents séquestrants, les agents plastifiants, les agents solubilisants, les agents acidifiants, des agents alcalinisants, les agents épaississants minéraux ou organiques, les agents antioxydants, les hydroxyacides, les parfums et les agents conservateurs.

31. Utilisation de la composition selon l'une quelconque des revendications 1 à 30, comme composition de traitement ou de soin cosmétique des matières kératiniques.

32. Utilisation de la composition selon l'une quelconque des revendications 1 à 30, comme shampooing ou composition à appliquer avant ou après un shampooing.

33. Procédé de traitement cosmétique, comprenant l'application sur les cheveux d'une quantité efficace d'une composition cosmétique selon l'une quelconque des revendications 1 à 30.

## Claims

1. Cosmetic composition for the care of keratin materials, comprising, in an aqueous medium:
- at least one silicone,
- at least one mono- or diester of fatty acid and of ethylene glycol or of polyethylene glycol, and
- oxyethylenated sorbitan monolaurate comprising 4 OE.

2. Composition according to the preceding claim, **characterized in that** it comprises at least 0.5% by weight of oxyethylenated sorbitan monolaurate comprising 4 OE, relative to the total weight of the composition.

3. Composition according to Claim 2, **characterized in that** it comprises from 0.5 to 10% by weight of oxyethylenated sorbitan monolaurate comprising 4 OE, more preferably from 2 to 9% by weight, relative to the total weight of the composition.

4. Composition according to any one of the preceding claims, **characterized in that** the silicone comprises one or more polyorganosiloxanes that are insoluble in said composition.

5. Composition according to the preceding claim, **characterized in that** it comprises at least one volatile polyorganosiloxane, chosen from those having a boiling point of between 60°C and 260°C, and more particularly from:
(i) cyclic silicones containing from 3 to 7 silicon atoms, and
(ii) linear volatile silicones having 2 to 9 silicon atoms and possessing a viscosity of less than or equal to 5 × 10⁻⁶ m²/s at 25°C.

6. Composition according to Claim 4, **characterized in that** it comprises at least one non-volatile polyorganosiloxane, chosen from polyalkylsiloxanes, polyarylsiloxanes, polyalkylarylsiloxanes, silicone gums and resins, polyorganosiloxanes modified with organofunctional groups, polysiloxane(A)-polyoxyalkylene(B) linear block copolymers of (A-B)ₙ type with n >3; grafted silicone polymers, with a non-silicone organic backbone, consisting of an organic main chain formed from organic monomers containing no silicone, onto which is grafted, within said chain and also, optionally, at at least one of its ends, at least one polysiloxane macromonomer; grafted silicone polymers, with a polysiloxane backbone grafted with non-silicone organic monomers, comprising a main chain of polysiloxane onto which is grafted, within said chain and also, optionally, at at least one of its ends, at least one organic macromonomer containing no silicone; and mixtures thereof.

7. Composition according to Claim 6, **characterized in that** the polyalkylsiloxanes are chosen from polydimethylsiloxanes.

8. Composition according to any one of the preceding claims, **characterized in that** it contains at least 0.1% by weight of silicone(s), relative to the total weight of the composition.

9. Composition according to Claim 6, **characterized in that** it contains from 0.1 to 20% by weight of silicone(s), more preferably from 0.5 to 20% by weight, even more preferably from 0.7 to 10% by weight, relative to the total weight of the composition.

10. Composition according to any one of the preceding claims, **characterized in that** said mono- or diester of a fatty acid and of ethylene glycol or of polyethylene glycol comprises a polyethylene glycol group, the latter comprising from 2 to 10 ethylene glycol units.

11. Composition according to any one of Claims 1 to 9, **characterized in that** said mono- or diester of fatty acid and of ethylene glycol or of polyethylene glycol is a mono- or diester of C₁₂ to C₂₄ fatty acid and of ethylene glycol.

12. Composition according to Claim 11, **characterized in that** said mono- or diester of fatty acid and of ethylene glycol or of polyethylene glycol is ethylene glycol distearate.

13. Composition according to any one of the preceding claims, **characterized in that** it contains at least 0.5% by weight of mono- or diester(s) of fatty acid and of ethylene glycol or of polyethylene glycol, relative to the total weight of the composition.

14. Composition according to the preceding claim, **characterized in that** it contains from 0.5 to 10% by weight, more preferably from 0.8 to 10% by weight, and even more preferably from 1 to 5% by weight of mono- or diester(s) of fatty acid and of ethylene glycol or polyethylene glycol, relative to the total weight of the composition.

15. Composition according to any one of the preceding claims, **characterized in that** it also comprises at least one monoester of a C₈ to C₂₄ fatty acid and of oxyethylenated sorbitan comprising from 15 to 50 oxyethylene units.

16. Composition according to the preceding claim, **characterized in that** said monoester is a monoester of a C₈ to C₂₄ fatty acid and of oxyethylenated sorbitan comprising 20 oxyethylene units, and preferably is oxyethylenated sorbitan monolaurate comprising 20 OE.

17. Composition according to either one of Claims 15 and 16, **characterized in that** it comprises from 0.1 to 10% by weight of monoester of a C₈ to C₂₄ fatty acid and of oxyethylenated sorbitan comprising from 15 to 50 oxyethylene units, preferably from 0.5 to 5% by weight, relative to the total weight of the composition.

18. Composition according to any one of the preceding claims, **characterized in that** it also comprises at least one anionic and/or nonionic and/or amphoteric surfactant.

19. Composition according to Claim 18, **characterized in that** it comprises at least one anionic surfactant and at least one amphoteric or zwitterionic surfactant.

20. Composition according to either one of Claims 18 and 19, **characterized in that** the anionic surfactant is chosen from alkyl sulphates, alkyl ether sulphates, and mixtures thereof.

21. Composition according to any one of Claims 18 to 20, **characterized in that** the amount of the anionic surfactant(s) ranges from 0.5 to 50% by weight, better still from 4 to 20% by weight relative to the total weight of the composition.

22. Composition according to any one of Claims 18 to 20, **characterized in that** the amphoteric or zwitterionic surfactant is chosen from (C₈-₂₀ alkyl)betaines, (C₈-₂₀ alkyl)amino(C₆₋₈ alkyl)betaines, and mixtures thereof.

23. Composition according to any one of the preceding claims, **characterized in that** it also comprises at least one cationic surfactant.

24. Composition according to any one of the preceding claims, **characterized in that** it also comprises at least one cationic polymer.

25. Composition according to Claim 24, **characterized in that** it comprises from 0.01 to 10% by weight of cationic polymer(s), preferably from 0.1 to 5% by weight, relative to the total weight of the composition.

26. Composition according to any one of the preceding claims, **characterized in that** it also comprises one or more anti-dandruff agent(s).

27. Composition according to Claim 26, **characterized in that** it comprises from 0.001 to 10% by weight of anti-dandruff agent(s), preferably from 0.1 to 5% by weight, relative to the total weight of the composition.

28. Composition according to any one of the preceding claims, **characterized in that** the aqueous medium consists of water or of a mixture of water and of at least one cosmetically acceptable solvent.

29. Composition according to Claim 28, **characterized in that** the solvent is chosen from C₁-C₄ lower alcohols and polyols.

30. Composition according to any one of the preceding claims, **characterized in that** it also comprises one or more conventional additives, such as anti-hairloss agents, oxidizing agents, ceramides and pseudo-ceramides, vitamins and provitamins including panthenol, plant, animal, mineral or synthetic oils, waxes, sunscreens, coloured or non-coloured inorganic or organic pigments, dyes, sequestering agents, plasticizers, solubilizing agents, acidifying agents, basifying agents, inorganic or organic thickeners, antioxidants, hydroxy acids, fragrances and preserving agents.

31. Use of the composition according to any one of Claims 1 to 30, as a composition for the cosmetic treatment or care of keratin materials.

32. Use of the composition according to any one of Claims 1 to 30, as a shampoo or a composition to be applied before or after a shampoo.

33. Cosmetic treatment process, comprising the application to the hair of an effective amount of a cosmetic composition according to any one of Claims 1 to 30.

## Patentansprüche

1. Kosmetische Zusammensetzung für die Pflege von Keratinmaterialien, die in einem wässerigen Milieu enthält:
- wenigstens ein Silikon,
- wenigstens einen Ethylenglykol- oder Polyethylenglykol-Mono- oder Di-Fettsäureester und
- Monolaurat von Oxyethylensorbitan, das 4 OE enthält.

2. Zusammensetzung nach dem vorhergehenden Anspruch, **dadurch gekennzeichnet, dass** sie wenigstens 0,5 Gew.-% Monolaurat von Oxyethylensorbitan mit 4 OE bezogen auf das Gesamtgewicht der Zusammensetzung enthält.

3. Zusammensetzung nach Anspruch 2, **dadurch gekennzeichnet, dass** sie 0,5 bis 10 Gew.-% Monolaurat von Oxyethylensorbitan mit 4 OE, bevorzugter 2 bis 9 Gew.-% bezogen auf das Gesamtgewicht der Zusammensetzung enthält.

4. Zusammensetzung nach einem der vorhergehenden Ansprüche, **dadurch gekennzeichnet, dass** das Silikon ein oder mehrere Polyorganosiloxane, die in der Zusammensetzung unlöslich sind, enthält.

5. Zusammensetzung nach dem vorhergehenden Anspruch, **dadurch gekennzeichnet, dass** sie wenigstens ein flüchtiges Polyorganosiloxan enthält, ausgewählt unter jenen, die einen Siedepunkt zwischen 60°C und 260°C besitzen und insbesondere unter
(i) zyklischen Silikonen mit 3 bis 7 Siliziumatomen und
(ii) linearen flüchtigen Silikonen mit 2 bis 9 Siliziumatomen und mit einer Viskosität kleiner oder gleich 5.10⁻⁶m²/s bei 25°C.

6. Zusammensetzung nach Anspruch 4, **dadurch gekennzeichnet, dass** sie wenigstens ein nicht-flüchtiges Polyorganosiloxan enthält, ausgewählt unter den Polyalkyl-siloxanen, Polyarylsiloxanen, Polyalkylarylsiloxanen, Silikongummi und -harzen, durch organofunktionelle Gruppen modifizierte Polyorganosiloxane, lineare Block-Copoly-mere Polysiloxan(A)-Polyoxyalkylen(B) der Art (A-B)ₙ mit n >3; gepropfte Silikon-polymere mit einem organischen, nicht-Silikon-Skelett, gebildet von einer organischen Hauptkette, gebildet ausgehend von organischen Monomeren, die kein Silikon enthalten, auf der sich aufgepfropft innerhalb der genannten Kette ebenso wie gegebenenfalls an wenigstens einem ihrer Enden, wenigstens ein makromonomeres Polysiloxan findet; gepfropfte Silikonpolymere mit Polysiloxanskelett gepfropft durch organische nicht-Silikon-Monomere enthaltend eine Hauptkette von Polysiloxan, auf der sich im Inneren der Kette ebenso wie gegebenenfalls an wenigstens einem ihrer Enden, wenigstens ein organisches Makromonomer gepfropft findet, das kein Silikon enthält, ebenso wie ihre Mischungen.

7. Zusammensetzung nach Anspruch 6, **dadurch gekennzeichnet, dass** die Polyalkylsiloxane ausgewählt sind unter den Polydimethylsiloxanen.

8. Zusammensetzung nach einem der vorhergehenden Ansprüche, **dadurch gekennzeichnet, dass** sie wenigstens 0,1 Gew.-% Silikon(e) bezogen auf das Gesamtgewicht der Zusammensetzung enthält.

9. Zusammensetzung nach Anspruch 6, **dadurch gekennzeichnet, dass** sie 0,1 bis 20 Gew.-% Silikon(e), bevorzugter 0,5 bis 20 Gew.-% und noch bevorzugter 0,7 bis 10 Gew.-% bezogen auf das Gesamtgewicht der Zusammensetzung enthält.

10. Zusammensetzung nach einem der vorhergehenden Ansprüche**, dadurch gekennzeichnet, dass** der genannte Ethylenglykol- oder Polyethylenglykol-Mono- oder Di-Fettsäureester eine Polyethylenglykolgruppierung enthält, welche letztere von 2 bis 10 Ethylenglykoleinheiten enthält.

11. Zusammensetzung nach einem der Ansprüche 1 bis 9, **dadurch gekennzeichnet, dass** der genannte Ethylenglykol- oder Polyethylenglykol-Mono- oder Di-Fettsäureester ein Mono- oder Diester von C₁₂ bis C₂₄ Fettsäure und Ethylenglykol ist.

12. Zusammensetzung nach Anspruch 11, **dadurch gekennzeichnet, dass** der genannte Ethylenglykol- oder Polyethylenglykol- Mono- oder Di-Fettsäureester das Ethylenglykoldistearat ist.

13. Zusammensetzung nach einem der vorhergehenden Ansprüche, **dadurch gekennzeichnet, dass** sie wenigstens 0,5 Gew.-% von Ethylenglykol- oder Polyethylenglykol-Mono- oder Di-Fettsäureester(n) bezogen auf das Gesamtgewicht der Zusammensetzung enthält.

14. Zusammensetzung nach dem vorhergehenden Anspruch, **dadurch gekennzeichnet, dass** sie 0,5 bis 10 Gew.-%, bevorzugter 0,8 bis 10 Gew.-% und noch bevorzugter 1 bis 5 Gew.-% von Ethylenglykol- oder Polyethylenglykol- Mono- oder Di-Fettsäure-ester(n) bezogen auf das Gesamtgewicht der Zusammensetzung enthält.

15. Zusammensetzung nach einem der vorhergehenden Ansprüche, **dadurch gekennzeichnet, dass** sie auch wenigstens einen Monoester einer C₈ bis C₂₄ Fettsäure und Oxyethylensorbitan enthaltend 15 bis 50 Oxyethyleneinheiten enthält.

16. Zusammensetzung nach dem vorhergehenden Anspruch, **dadurch gekennzeichnet, dass** der Monoester ein Monoester einer C₈ bis C₂₄ Fettsäure und Oxyethylensorbitan enthaltend 20 Oxyethyleneinheiten, vorzugsweise Sorbitanmonolaurat von Oxyethylen-sorbitan mit 20 OE ist.

17. Zusammensetzung nach einem der Ansprüche 15 und 16, **dadurch gekennzeichnet, dass** sie 0,1 bis 10 Gew.-% Monoester der C₈ bis C₂₄ Fettsäure und Oxyethylen-sorbitan enthaltend 15 bis 50 Oxyethyleneinheiten, vorzugsweise 0,5 bis 5 Gew.-% bezogen auf das Gesamtgewicht der Zusammensetzung, enthält.

18. Zusammensetzung nach einem der vorhergehenden Ansprüche, **dadurch gekennzeichnet, dass** sie auch wenigstens einen anionischen und/oder nicht-ionischen und/oder amphoteren oberflächenaktiven Wirkstoff enthält.

19. Zusammensetzung nach Anspruch 18, **dadurch gekennzeichnet, dass** sie wenigstens einen anionischen oberflächenaktiven Wirkstoff und wenigstens einen amphoteren oder zwitterionischen oberflächenaktiven Wirkstoff enthält.

20. Zusammensetzung nach einem der Ansprüche 18 oder 19, **dadurch gekennzeichnet, dass** der anionische oberflächenaktive Wirkstoff ausgewählt ist unter Alkylsulfaten, Alkyläthersulfaten und ihren Mischungen.

21. Zusammensetzung nach einem der Ansprüche 18 bis 20, **dadurch gekennzeichnet, dass** die Menge des oder der oberflächenaktiven anionischen Wirkstoffe im Intervall von 0,5 bis 50 Gew.-%, besser noch von 4 bis 20 Gew.-%, bezogen auf das Gesamtgewicht der Zusammensetzung liegt.

22. Zusammensetzung nach einem der Ansprüche 18 bis 20, **dadurch gekennzeichnet, dass** der amphotäre oder zwitterionische oberflächenaktive Wirkstoff unter den (C₈₋₂₀ Alkyl)betainen, den (C₈₋₂₀ Alkyl)amido(C₆₋₈ alkyl)betainen und ihren Mischungen ausgewählt ist.

23. Zusammensetzung nach einem der vorhergehenden Ansprüche, **dadurch gekennzeichnet, dass** sie überdies wenigstens einen kationischen oberflächenaktiven Wirkstoff enthält.

24. Zusammensetzung nach einem der vorhergehenden Ansprüche, **dadurch gekennzeichnet, dass** sie überdies wenigstens ein kationisches Polymer enthält.

25. Zusammensetzung nach Anspruch 24, **dadurch gekennzeichnet, dass** sie von 0,01 bis 10 Gew.-% kationisches Polymer/kationische Polymere, vorzugsweise 0,1 bis 5 Gew.-%, bezogen auf das Gesamtgewicht der Zusammensetzung, enthält.

26. Zusammensetzung nach einem der vorhergehenden Ansprüche, **dadurch gekennzeichnet, dass** sie überdies ein oder mehrere Antischuppenmittel enthält.

27. Zusammensetzung nach Anspruch 26, **dadurch gekennzeichnet, dass** sie von 0,001 bis 10 Gew.-% Antischuppenmittel, vorzugsweise 0,1 bis 5 Gew.-%, bezogen auf das Gesamtgewicht der Zusammensetzung, enthält.

28. Zusammensetzung nach einem der vorhergehenden Ansprüche, **dadurch gekennzeichnet, dass** das wässerige Milieu aus Wasser oder aus einer Mischung aus Wasser und wenigstens einem kosmetisch annehmbaren Lösungsmittel gebildet ist.

29. Zusammensetzung nach Anspruch 28, **dadurch gekennzeichnet, dass** das Lösungsmittel ausgewählt ist unter niedrigen C₁ bis C₄ Alkoholen und Polyolen.

30. Zusammensetzung nach einem der vorhergehenden Ansprüche, **dadurch gekennzeichnet, dass** sie überdies einen oder mehrere herkömmliche Zusatzstoffe enthält, wie Mittel gegen Haarausfall, Oxydationsmittel, Ceramide und Pseudoceramide, Vitamine und Provitamine, darunter Panthenol, pflanzliche Öle, tierische Öle, Mineralöle oder synthetische Öle, Wachse, Ceramide und Pseudoceramide, Sonnenfilter, anorganische oder organische, gefärbete oder nicht-gefärbte Pigmente, Färbemittel, Sequestriermittel, Plastifiziermittel, Solubilisiermittel, Säuerungsmittel, Alkalisierungsmittel, anorganische oder organische Verdickungsmittel, Antioxidantien, Hydroxysäuren, Parfüms und Konservierungsmittel.

31. Verwendung der Zusammensetzung nach einem der Ansprüche 1 bis 30 als Zusammensetzung für die Behandlung oder die kosmetische Pflege von Keratinmaterialien.

32. Verwendung der Zusammensetzung nach einem der Ansprüche 1 bis 30 als Shampoo oder als Zusammensetzung zum Auftragen vor oder nach dem Shampoonieren.

33. Verfahren zum kosmetischen Behandeln, umfassend die Anwendung einer wirksamen Menge einer kosmetischen Zusammensetzung gemäß irgendeinem der Ansprüche 1 bis 30 auf Haare .
